Europäisches Patentamt

European Patent Office

Office européen des brevets

(19)

(11) Veröffentlichungsnummer: **0 144 483**

**A2**

(12) # EUROPÄISCHE PATENTANMELDUNG

(21) Anmeldenummer: 84100711.5

(22) Anmeldetag: 24.01.84

(51) Int. Cl.⁴: **A 61 M 5/00**

(30) Priorität: 09.12.83 DE 8335341 U

(43) Veröffentlichungstag der Anmeldung:
19.06.85 Patentblatt 85/25

(84) Benannte Vertragsstaaten:
AT BE CH DE FR GB IT LI LU NL SE

(71) Anmelder: Arzneimittel GmbH Apotheker Vetter & Co.
Ravensburg
Marienplatz 79-81
D-7980 Ravensburg(DE)

(72) Erfinder: Geprägs, Peter
Scherzachstrasse 5
D-7981 Schlier(DE)

(74) Vertreter: Fay, Hermann, Dipl.-Phys. Dr.
Ensingerstrasse 21 Postfach 1767
D-7900 Ulm (Donau)(DE)

(54) Spritze für medizinische Zwecke.

(57) Der Spritzenzylinder (1) trägt an einem nadelseitigen Zylindermundstück (2) ein axial von außen aufgesetztes und durch eine Ringkappe (4) gehaltenes Nadelansatzstück (3). Zylindermundstück (2) und Nadelansatzstück (3) besitzen je einen Außenbund (5, 6), die von der sie übergreifenden Ringkappe (4) axial gegeneinander verpreßt sind. Das Zylindermundstück (2) besitzt eine sich nadelseitig erweiternde und bis zur nadelseitigen Stirnfläche (10) seines Außenbundes (6) erstreckende konische Innenfläche (11) und das Nadelansatzstück (3) einen der konischen Innenfläche (11) anliegende Konusteil (12). Die den Außenbund (5) des Nadelansatzstückes (3) mit einem Ringflansch (13) übergreifende Ringkappe (4) ist am Zylindermundstück (2) mit einer die zylinderseitige Stirnfläche (14) seines Außenbundes (6) übergreifenden Innenschulter (15) elastisch eingerastet.

*Fig. 2*

Arzneimittel GmbH
Apotheker Vetter & Co. Ravensburg
Marienplatz 79-81
7980 Ravensburg

7900 Ulm, 13.01.84
Akte E/6150 sr

<u>Spritze für medizinische Zwecke</u>

Die Erfindung betrifft eine Spritze für medizinische
Zwecke mit einem mindestens einen verschiebbaren
Spritzenkolben aufweisenden Spritzenzylinder, der an
einem nadelseitigen Zylindermundstück ein axial von
außen aufgesetztes und durch eine Ringkappe gehaltenes Nadelansatzstück trägt, wobei das Zylindermundstück und das Nadelansatzstück je einen Außenbund
aufweisen und beide Außenbunde von der sie übergreifenden Ringkappe axial gegeneinander verpreßt sind.

Bei bekannten Spritzen dieser Art, insbes. Einmalspritzen, ist zwischen beide Außenbunde eine Dichtringscheibe eingelegt und mittels der Ringkappe verpreßt, die aus Metall besteht und über die voneinander abgewandten Stirnseiten der beiden Außenbunde
umgebogen bzw. umgebördelt ist. Dabei kann eine hohe
axiale Anpreßkraft aufgebracht und durch die umgebördelte Ringkappe aufrecht erhalten werden, so daß die
Dichtringscheibe eine gute Abdichtung ergibt. Diese
Montage der Ringkappe erfolgt bei noch leerer Spritze,
ist also ohne weiteres für Spritzen geeignet, die,
wie Einmalspritzen, durch das hintere Ende des Spritzen-

0144483

- 2 -

zylinders bei zunächst fehlendem Spritzenkolben oder die in üblicher Weise durch Aufziehen der Injektionsflüssigkeit durch die Injektionsnadel mittels des Spritzenkolbens befüllt werden. Eine Befüllung der Spritze vom nadelseitigen Ende her bei zunächst noch fehlendem Nadelansatzstück ist nicht möglich, weil die Montage des Nadelansatzstückes mittels der Ringkappe bei bereits befüllter Spritze mit Schwierigkeiten verbunden wäre.

Der Erfindung liegt die Aufgabe zugrunde, eine Spritze der eingangs genannten Art so auszubilden, daß das Nadelansatzstück und die Ringkappe auch bei schon befüllter Spritze unschwer montiert werden können und sichere Dichtheit des Spritzenzylinderabschlusses gewährleisten.

Diese Aufgabe wird nach der Erfindung dadurch gelöst, daß das Zylindermundstück eine sich nadelseitig erweiternde und bis zur nadelseitigen Stirnfläche seines Außenbundes erstreckende konische Innenfläche und das Nadelansatzstück einen der konischen Innenfläche anliegenden Konusteil aufweist, wobei der Durchmesser des Konusteils bei unverformtem freiem Nadelansatzstück etwas größer als der jeweils entsprechende Durchmesser der konischen Innenfläche ist, und daß die den Außenbund des Nadelansatzstückes mit einem Ringflansch übergreifende Ringkappe am Zylindermundstück mit einer die zylinderseitige Stirnfläche seines Außenbundes übergreifenden Innenschulter elastisch eingerastet ist. Dabei kann das Nadelansatzstück im Ganzen aus einem gummielastischen Werkstoff (Gummi oder elastomerer Kunststoff) bestehen, in den die Injektionsnadel
einvulkanisiert oder temperaturbeständig eingeklebt
sein kann. Die Injektionsnadel kann aber auch zunächst
überhaupt fehlen und erst unmittelbar vor der Applikation der Spritze in oder auf das Nadelansatzstück gesetzt werden, wozu am Nadelansatzstück in üblicher Weise ein Luerkonus angeformt sein kann, der sich übrigens
auch deswegen schon empfiehlt, weil er zum Aufstecken
einer die im Nadelansatzstück fest einvulkanisierte
oder eingeklebte Injektionsnadel schützenden Kappe dienen kann. Wird die Injektionsnadel erst unmittelbar vor
der Applikation der Spritze auf das Nadelansatzstück
aufgesteckt, kann der im Nadelansatzstück den Austritt
des Injektionsmittels ermöglichende Kanal durch eine
Verschlußkappe nach Art einer sogenannten Tip-Cap verschlossen sein, die auf dem Luerkonus sitzt und einen
in den Kanal eingreifenden und ihn abdichtenden zentralen Stift besitzt.

Der durch die Erfindung erreichte Fortschritt besteht
im wesentlichen darin, daß die Ringkappe nicht mehr
als Metallteil durch Biege- und Bördelungsprozesse
montiert werden muß, sondern als ein in der Regel aus
Kunststoff bestehendes Montageteil lediglich über das
dem Spritzenmundstück aufgesteckte Nadelansatzstück
gesteckt und bis zum Einrasten am Spritzenmundstück
angedrückt zu werden braucht. Dazu bedarf es wesentlich geringerer Andruckkräfte als sie bei einer Ringkappe aus Metall zum Verpressen beider Außenbunde über
die Dichtringscheibe erforderlich sind, um die gewünschte Dichtigkeit sicher zu stellen. Denn bei der Erfindung wird die axiale Andruckkraft über den Kegelwinkel des Konusteils in eine hohe Anpreßkraft zwischen der Umfangsfläche des Konusteils des Nadelansatzstückes und der konischen Innenfläche des Zylindermundstückes transformiert, so daß die durch das Einrasten erreichte axiale Andruckkraft zur sicheren Abdichtung ausreicht. Im übrigen ermöglicht das durch die konische Innenfläche im Vergleich zur Innenweite des Spritzenzylinders selbst nur wenig verengte Zylindermundstück die Befüllung von Einmalspritzen vom nadelseitigen Ende her, was besonders dann wichtige Bedeutung erhält, wenn die Spritze für Lyophilisate bestimmt ist und die Lyophilisierung in der Spritze selbst und durch das nadelseitige Ende des Spritzenzylinders hindurch erfolgen soll. Unabhängig davon kann die Spritze mit eingerastetem Nadelansatzstück unter hohen Temperaturen behandelt werden, was sowohl ihre Sterilisierung im Durchlauftunnel mit dem Vorteil eines kontinuierlichen Bearbeitungsablaufes (im Gegensatz zum unterbrochenen Ablauf bei Sterilisierung im Autoklaven) als auch ihre Silikonisierung durch Einbrennen ermöglicht.

Eine bevorzugte Ausführungsform der Erfindung ist dadurch gekennzeichnet, daß sich im Zylindermundstück zylinderseitig an die konische Innenfläche eine zylindrische Innenfläche anschließt, der ein Zylinderteil des Nadelansatzstückes anliegt, wobei der Durchmesser des Zylinderteils bei freiem unverformten Nadelansatzstück ebenfalls etwas größer ist als der Innendurchmesser der zylindrischen Innenfläche, so daß sich zwischen dem Zylinderteil des Nadelansatzstückes und der zylindrischen Innenfläche des Zylindermundstückes durch die Kompression des Zylinderteils bei in das Zylindermundstück eingesetztem Nadelansatzstück eine zusätzliche Abdichtung zwischen dem Nadelansatzstück und dem Zylindermundstück ergibt. Es genügt dann in weiterer Ausbildung der Erfindung, daß die beiden Außenbunde mit ihren einander zugewandten Stirnflächen unmittelbar aneinander liegen, wobei die Stirnfläche des Außenbundes am Zylindermundstück als sehr flache, in Richtung zur Nadel zusammenlaufende Kegelfläche ausgebildet ist, so daß sich der höchste Dichtungsdruck unmittelbar am Rande der Mündung der konischen Innenfläche in der Stirnfläche des Außenbundes des Zylindermundstücks einstellt. Auf eine besondere Dichtringscheibe wie bei den bekannten Spritzen kann dann verzichtet werden. Zweckmäßig sind im Nadelansatzstück zwischen dem Zylinderteil, dem Konusteil und dem Außenbund schmale Ringnuten vorgesehen, mit welchen es möglich ist, die beim Einsetzen und Verspannen des Nadelansatzstückes im bzw. am Zylindermundstück entstehenden Werkstoffverdrängungen des Nadelansatzstückes, insbes. im Bereich seines Zylinder- und Konusteils, in jeweils gewünschter Weise zu steuern, so daß sich die unmittelbaren Anpreßkräfte zwischen den aneinander liegenden Flächen des Zylinder- und Konusteils einerseits und des Zylindermundstücks andererseits weitgehend unabhängig voneinander einstellen lassen. Dem gleichen Zweck kann eine in der zylinderseitigen Stirnfläche des Zylinderteils des Nadelansatzstückes vorgesehene flache hohlkegelförmige Aussparung dienen, über die es möglich ist, die Werkstoffverdrängung insbes. im Zylinderteil des Nadelansatzstückes zu beeinflussen. Im übrigen empfiehlt es sich, daß die Innenschulter der Ringkappe von einem Rastring gebildet ist, der an seinem zylinderseitigen Innenrand mit einer sich zum Spritzenzylinder hin öffnenden Kegelfläche ausgestattet ist, die es in einfacher Weise ermöglicht, den Rastring über die Außenbunde hinweg zu führen und im dazu erforderlichen Umfang elastisch aufzuweiten.

Im folgenden wird die Erfindung an einem in der Zeichnung dargestellten Ausführungsbeispiel näher erläutert; es zeigen:

Fig.1 eine erfindungsgemäße Spritze mit lediglich schematisch angedeutetem Spritzenkolben im Axialschnitt,

Fig. 2 das vordere Ende des Spritzenzylinders und des Nadelansatzstückes ohne Injektionsnadel im nicht zusammengesetzten Zustand in gegenüber Fig. 1 vergrößerter Darstellung.

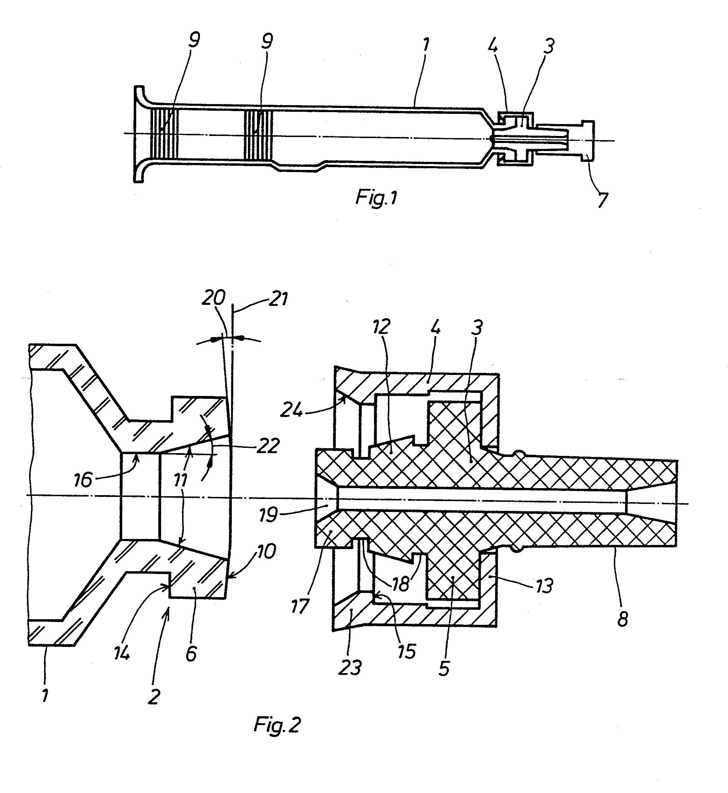

In der Zeichnung ist eine Zweikammerspritze mit zwei Spritzenkolben 9 dargestellt und der Spritzenzylinder mit 1, sein Zylindermundstück mit 2 und das dem Zylindermundstück axial von außen aufsetzbare Nadelansatzstück mit 3 bezeichnet. Das Nadelansatzstück 3 wird durch eine Ringkappe 4 gehalten, wozu das Zylindermundstück 2 und das Nadelansatzstück 3 je einen Außenbund 5, 6 aufweisen und beide Außenbunde 5,6 von der sie übergreifenden Ringkappe 4 axial gegeneinander verpreßt sind. Das Nadelansatzstück 3 kann, soweit an ihm noch keine Injektionsnadel montiert ist, entsprechend Fig. 1 durch ein Tip-Cap 7 verschlossen sein.

Ist in das Nadelansatzstück 3 eine Injektionsnadel eingesetzt, kann auf dem in Fig. 1 die Tip-Cap 7 aufnehmenden Luerkonus 8 des Nadelansatzstückes eine in der Zeichnung nicht dargestellte Nadelschutzkappe aufgesteckt werden. Das mit dem Spritzenzylinder 1 einstückige Zylindermundstück 2 besitzt eine sich nadelseitig erweiternde und bis zur nadelseitigen Stirnfläche 10 seines Außenbundes 6 erstreckende konische Innenfläche 11. Das aus Gummi oder einem elastomeren Kunststoff bestehende Nadelansatzstück 3 besitzt einen Konusteil 12, der bei am Zylindermundstück 2 fertig montiertem Nadelansatzstück 3 dessen konischer Innenfläche 11 anliegt. Der Durchmesser des Konusteils 12 ist bei noch nicht montiertem, also unverformtem freiem Nadelansatzstück 3 etwas größer als der jeweils entsprechende Durchmesser der konischen Innenfläche 11, so daß sich zwischen der Umfangsfläche des Konusteils 12 und der zugeordneten konischen Innenfläche 11 des Zylindermundstücks 2 eine hohe Pressung ergibt. Die den Außenbund 5 des Nadelansatzstückes 3 mit einem Ringflansch 13 übergreifende Ringkappe 4 aus Kunststoff ist am Zylindermundstück 2 mit einer die zylinderseitige Stirnfläche 14 seines Außenbundes 6 übergreifenden Innenschulter 15 elastisch eingerastet. Im Zylindermundstück 2 schließt sich zylinderseitig an die konische Innenfläche 11 eine zylindrische Innenfläche 16 an, der am Nadelansatzstück 3 ein Zylinderteil 17 entspricht, das bei am Zylindermundstück 2 montiertem Nadelansatzstück 3 mit seiner zylindrischen Außenfläche der zylindrischen Innenfläche 16 des Zylindermundstücks 2 anliegt. Auch hier ist der Durchmesser des Zylinderteils 17 bei noch nicht montiertem, also freiem unverformten Nadelansatzstück 3 etwas größer als der Innendurchmesser der zylindrischen Innenfläche 16, so daß die Anpreßkraft der einander anliegenden Zylinderflächen des Zylinderteils 17 einerseits und des Zylindermundstücks 2 andererseits gewährleistet ist. Bei dem Verpressen des Zylinderteils 17 und des Konusteils 12 im Zylindermundstück 2 finden im Nadelansatzstück 3 jedenfalls im Bereich des Zylinderteils 17 und des Konusteils 12 Werkstoffverdrängungen statt, die in besonders einfacher Weise dadurch gesteuert werden können, daß im Nadelansatzstück 3 zwischen dem Zylinderteil 17, dem Konusteil 12 und dem Außenbund 5 schmale Ringnuten 18 vorgesehen sind. Aus dem gleichen Grund kann sich in der zylinderseitigen Stirnfläche des Zylinderteils 17 des Nadelansatzstückes 3 eine flache hohlkegelförmige Aussparung 19 befinden. Die beiden Außenbunde 5, 6 liegen mit ihren einander zugewandten Stirnflächen unmittelbar aneinander. Die Stirnfläche 10 des Außenbundes 6 am Zylindermundstück 2 ist als sehr flache, in Richtung zur Nadel zusammenlaufende Kegelfläche ausgebildet, die im Ausführungsbeispiel einen Winkel 20 von 5° mit der zur Spritzenachse senkrechten Mündungsebene 21 bildet. Der Konuswinkel 22 der konischen Innenfläche 11 des Zylindermundstückes 2 und entsprechend des Zylinderteils 12 am Nadelansatzstück 3 beträgt 15°. Die Innenschulter 15 der Ringkappe 4 ist von einem Rastring 23 gebildet, der an seinem zylinderseitigen Innenrand mit einer sich zum Spritzenzylinder 1 hin öffnenden Kegelfläche 24 ausgestattet ist, die es in einfacher Weise ermöglicht, beim Aufstecken der Ringkappe 4 sowohl auf den Außenbund 5 des Nadelansatzstückes 3 als auch beim Aufstecken auf den Außenbund 6 des Zylindermundstückes 2 den Rastring 23 in einfacher Weise im erforderlichen Umfang elastisch aufzuweiten.

Arzneimittel GmbH
Apotheker Vetter & Co. Ravensburg
Marienplatz 79-81
7980 Ravensburg

7900 Ulm, 13.01.84
Akte  E/6150 sr

Schutzansprüche:

1. Spritze für medizinische Zwecke mit einem mindestens einen verschiebbaren Spritzenkolben aufweisenden Spritzenzylinder, der an einem nadelseitigen Zylindermundstück ein axial von außen aufgesetztes und durch eine Ringkappe gehaltenes Nadelansatzstück trägt, wobei das Zylindermundstück und das Nadelansatzstück je einen Außenbund aufweisen und beide Außenbunde von der sie übergreifenden Ringkappe axial gegeneinander verpreßt sind, dadurch gekennzeichnet, daß das Zylindermundstück (2) eine sich nadelseitig erweiternde und bis zur nadelseitigen Stirnfläche (10) seines Außenbundes (6) erstreckende konische Innenfläche (11) und das Nadelansatzstück (3) einen der konischen Innenfläche (11) anliegenden Konusteil (12) aufweist, wobei der Durchmesser des Konusteils (12) bei unverformtem freien Nadelansatzstück (3) etwas größer als der jeweils entsprechende Durchmesser der konischen Innenfläche (11) ist, und daß die den Außenbund (5) des Nadelansatzstückes (3) mit einem Ringflansch (13) übergreifende Ringkappe (4) am Zylindermundstück (2) mit einer die zylinderseitige Stirnfläche (14) seines Außenbundes (6) übergreifenden Innenschulter (15) elastisch eingerastet ist.

• • •

Adresse: 79 Ulm/Donau,          Telefon          Telegramme          Postscheckkonto          Bankkonto
Postfach 1209, Ensingerstraße 21  (07 31) 6 49 46  Prosnat Ulmdonau      Stuttgart 732 37          Deutsche Bank AG Ulm 184 671

- 2 -

2. Spritze nach Anspruch 1, dadurch gekennzeichnet, daß sich im Zylindermundstück (2) zylinderseitig an die konische Innenfläche (11) eine zylindrische Innenfläche (16) anschließt, der ein Zylinderteil (17) des Nadelansatzstückes (3) anliegt, wobei der Durchmesser des Zylinderteils (17) bei freiem unverformten Nadelansatzstück (3) etwas größer ist als der Innendurchmesser der zylindrischen Innenfläche (16).

3. Spritze nach Anspruch 1 oder 2, dadurch gekennzeichnet, daß die beiden Außenbunde (5, 6) mit ihren einander zugewandten Stirnflächen unmittelbar aneinander liegen, wobei die Stirnfläche (10) des Außenbundes (6) am Zylindermundstück (2) als sehr flache, in Richtung zur Nadel zusammenlaufende Kegelfläche ausgebildet ist.

4. Spritze nach den Ansprüchen 1 bis 3, dadurch gekennzeichnet, daß im Nadelansatzstück (3) zwischen dem Zylinderteil (17), dem Konusteil (12) und dem Außenbund (5) schmale Ringnuten (18) vorgesehen sind.

5. Spritze nach einem der Ansprüche 1 bis 4, dadurch gekennzeichnet, daß die Innenschulter (15) der Ringkappe (4) von einem Rastring (23) gebildet ist, der an seinem zylinderseitigen Innenrand mit einer sich zum Spritzenzylinder (1) hin öffnenden Kegelfläche (24) ausgestattet ist.

6. Spritze nach Anspruch 2, dadurch gekennzeichnet, daß in der zylinderseitigen Stirnfläche des Zylinderteils (17) des Nadelansatzstückes (3) eine flache hohlkegelförmige Aussparung (19) vorgesehen ist.

1/1

0144483

Fig.1

Fig.2